# EUROPEAN PATENT APPLICATION

(11) **EP 2 127 547 A2**
(43) Date of publication of application: **02.12.2009**
(21) Application number: 09170926.1
(22) Date of filing: 21.02.2006
(51) Int. Cl.: A41D 19/00, A61B 19/04

(54) **Glove with anti-roll down cuff**

(30) Priority: 22.03.2005 GB 0505862; 13.06.2005 GB 0511993
(62) Divisional of application: 06709844.2
(71) Applicant: Regent Medical Limited, Irlam, Manchester M44 5BJ (GB)
(72) Inventor: Day, Jonathan, Irlam, Greater Manchester M44 5BJ (GB); Leeming, Christine, Irlam, Greater Manchester M44 5BJ (GB); Rogers, Jan, Irlam, Greater Manchester M44 5BJ (GB); Williams, Haydn, Irlam, Greater Manchester M44 5BJ (GB)
(74) Representative: Hedges, Martin Nicholas

(57) **Abstract**

A glove comprises a hand portion 1 and a wrist portion 2 extending from the hand portion 1 and terminates in a hand insertion opening 3. The wrist portion 2 includes anti-roll down means in the form of a band of adhesive material 4 provided on a surface which, in use, will form the inside of the glove, whereby, in use, the adhesive portion 4 may be pressed against the cuff of a garment worn by the user so as to adhere the wrist portion of the glove thereto, thereby preventing roll-down of the glove.

## Description

The present invention relates to surgical gloves and the like and in particular to such gloves including anti roll down measures provided on the cuff of the glove.

Surgical gloves are designed with a smooth / slippery inner surface with the aim of making it easier for the wearer to don the glove. The gloves are designed to be worn over surgical gowns which were typically cotton based, but recently there have been significant advancements in the material for such gowns, including improvement in the impermeability of the materials achieved by treating the material with a hydrophobic moiety that improves the moisture repellent characteristics of the material. The material from which surgical gloves are typically made has a tacky, adhesive quality, and in order to facilitate the fitting and removal of the gloves onto and from the hands of the user, the formed glove is treated with a material which smoothes the surface thereof. However, an ongoing problem with existing glove and gown designs is that the smooth surfaces of the gloves give rise to a tendency for the cuff of the glove to move down the gown during use, exposing the woven cuff of the gown and hence presenting a risk of strike through of potentially infectious material.

In order to overcome this problem, gloves have been designed having textured wrist portions which are moulded into the material of the glove during the glove moulding process. For example, US-A-4095293 discloses a moulded glove having a plurality of longitudinal channels moulded around the circumference of the wrist portion of the glove together with a plurality of circumferential grooves in the region of the mouth of the glove which operate to prevent roll down. However, this arrangement has the problem that the moulding of the grooves into the glove causing a thinning of material around the wrist and cuff. Furthermore, due to the manner in which the grooves are moulded and the fact that the glove is turned inside out upon removal from the former, the grooves will end up on the outside of the glove and the glove hence needs to be reinverted prior to use in order for the grooves to perform their gripping function.

According to the present invention there is provided a glove, in particular a surgical glove, comprising a hand portion and a wrist portion extending from the hand portion and terminating in a hand insertion opening, the wrist portion including a region of adhesive material provided on a surface which, in use, will form the inside of the glove, whereby, in use, the adhesive portion may be pressed against the cuff of a garment worn by the user so as to adhere the wrist portion of the glove thereto, thereby preventing roll-down of the glove.

A glove in accordance with the invention has the advantage that it provides an effective and reliable system for preventing roll down of the wrist portion of surgical gloves in a manner which also provides an additional barrier to prevent the ingress of matter between the wrist portion of the glove and the outer surface of the surgical gown.

Preferably, the adhesive material extends around the inner circumference of the wrist portion of the glove which may be in a band, so as, in use, to secure the glove to the cuff of the garment around the entire periphery thereof. This has the advantage of anchoring the glove to the garment in a particularly secure and effective manner.

The adhesive may be a pressure sensitive adhesive that displays a surface of moderate tack or no tack prior to its activation. The adhesive may also be microencapsulated. Once the glove is donned over the gown, the user applies pressure around the cuff area, which causes activation of the adhesive agent, thereby rendering the cuff area tacky to an extent which enables adhesion of the gown.

The adhesive itself may be overlaid with a specific liner or release material which protects the adhesive, preventing it from sticking until required and from becoming polluted with dust, dirt etc, which might otherwise diminish its adhesiveness, until ready for use whilst having a low adhesion to the adhesive so as to be easily removable therefrom. The liner is then removed by the user once the glove has been fitted so as to expose the adhesive for fastening to the cuff of the gown.. The liner material advantageously has identical or similar elastomeric properties to those of the glove so that it expands and contracts with the glove.

The adhesive strip may be formed of material which is separate from and applied to the surface of the glove after it has been formed and finished with a suitable finishing material. For example, prior to its application to the glove, the adhesive itself may be bound to a suitable backing material displaying similar elastomeric properties to those of the glove, so as to form an adhesive tape. Alternatively, the adhesive may be applied as either a solution, a gel, or in the molten state, with liner material or the like applied thereover as required to protect the adhesive until ready for use.

Alternatively, the adhesive strip may be formed by an un-finished region of glove material whose tackiness has been maintained. In particular, an adhesive tape may be applied to an exposed region of un-finished glove material, so as to mask the underlying portion of the glove which is still tacky following the forming or moulding operation. The exposed areas of the glove are then finished to provide it with a less tacky surface - adhesive tape maintaining the tackiness of the underlying portion of the glove by preventing its exposure to the finishing material. Subsequent removal of the adhesive tape thus exposes a portion of the glove that has been shielded from the finishing operation and its tackiness is thereby maintained.

The present invention thereby further provides a method of manufacture of a surgical glove comprising the steps of coating a former with material to form the glove, applying masking material to the surface of the glove in the region of a wrist portion thereof, and carrying out a finishing operation on the glove which produces a smooth surface thereof, the masking material shielding the underlying glove material from the finishing operation so as to maintain the tacky and/or adhesive characteristic thereof.

Preferably the masking or adhesive material is applied in a band which extends around the entire circumference of the wrist portion and is located on a surface which, in use, forms an inside surface of the glove, the glove, in use, overlapping the cuff of the gown on the outside.

According to another aspect of the present invention there is provided a glove, in particular a surgical glove, comprising a hand portion and a wrist portion extending from the hand portion and terminating in a hand insertion opening, wherein the wrist portion of the glove, at least in the region of said opening, is reinforced with elastomeric material applied to a surface which, in use, will form an inside surface of the glove.

The present invention further provides a method of manufacturing a glove, in particular a surgical glove, comprising the steps of coating a former with material to form the glove, and selectively applying additional elastomeric material to areas of the glove material on the former which form a cuff portion of the glove so as to form reinforcing and gripping means on said cuff portion.

A glove and method of manufacture thereof in accordance with the invention has the advantage that the additional elastomeric material applied to the surface of the glove in the region of the cuff operates effectively to reduce roll down of the cuff whilst maintaining the strength and integrity of the glove.

The elastomeric material may be applied in a number of different patterns. Longitudinal or spiral bands extending from the cuff opening are particularly effective, but other banding arrangements are also possible, as are patterns of dots and the like.

Preferably, the elastomeric reinforcing material is the same material as used to form the glove itself, in particular latex, and is preferably applied to a thickness of 1mm, although other thicknesses and materials are also possible. Thickened elastomeric material is, in particular, sprayed or extruded onto the glove in the required pattern when the glove is on the former.

In order that the invention may be well understood, there will now be described some embodiment thereof, given by way of example, reference being made to the accompanying drawings, in which:
Figure 1 is a perspective view of a surgical glove according to a first embodiment of the invention;
Figure 2 is a perspective view of a glove, turned inside out, according to a second embodiment of the invention; and
Figure 3 is a perspective view of a glove, turned inside out, according to a third embodiment of the invention.

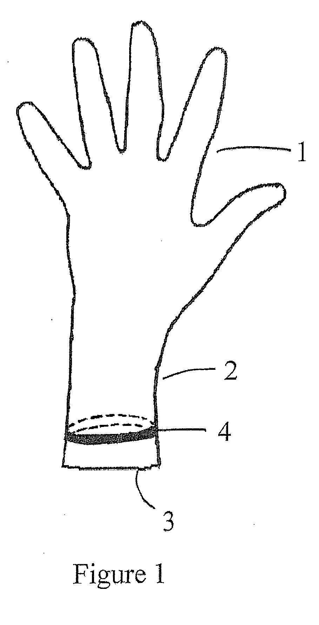

A surgical glove according to the invention as shown in Figure 1 comprises integrally formed hand 1 and cuff 2 portions, the cuff portion terminating in a hand opening 3 by means of which a user may insert a hand into the glove. The glove is manufactured according to normal procedures familiar to those experienced in the art using a former or mould onto which elastomeric material, such as latex, is straight or coagulant dipped and dried to form a film which is subsequently peeled off of the mould. Provided on the cuff portion 2 of the glove is a band of adhesive material 4 which extends around the circumference of the cuff 2, substantially parallel and proximate to the hand opening 3.

In one embodiment, the band of adhesive material is formed from the material of the glove itself by applying to the tacky surface of the formed but unfinished glove, in the region of the hand opening, a circumferential band of adhesive tape. This band of adhesive tape operates to mask the underlying portion of the glove during a subsequent coating process whereby a suitable slip coating is applied to the glove and / or during an operation in which the surfaces of the glove are treated, for example chemically and / or with a non powdered material the result of which produces a smooth finish thereon. By masking the underlying band of glove material, the tackiness of this portion of material is maintained since it not subjected to the coating or finishing processes. The adhesive tape then protects the underlying portion of material until the glove is ready for use, whereupon the user fits the glove onto the hand with the adhesive tape on the inner surface and the cuff portion positioned to overlap the outer surface of the cuff of the gown worn by the user. The adhesive tape is then removed by the user so as to expose the underlying tacky glove material, which is then pressed against the outer surface of the gown, adhering thereto and hence securing the cuff of the glove in position in a manner which prevents roll-down thereof.

In an alternative embodiment, the band of adhesive is formed by a separate material that is applied to the surface of the glove following the finishing step. According to this embodiment, a glove is made by the steps of providing a former shaped to the desired shape of the glove, coating the former, for example by dipping, with a thin layer of elastomeric material to form the glove. A finishing material is applied to both the inner and outer surface of the glove in order to produce a smooth surface. Next, a circumferential band of adhesive, potentially overlaid with a liner material, is applied to the glove in the region of the hand opening. The user then fits the glove onto the hand with the adhesive on the inner surface and the glove positioned to overlap the outer surface of the cuff of the gown worn by the user. The adhesive liner material, if present, is then removed by the user to expose the underlying adhesive which is then pressed against the outer surface of the gown, adhering thereto and hence securing the cuff of the glove in a manner that prevents roll-down thereof.

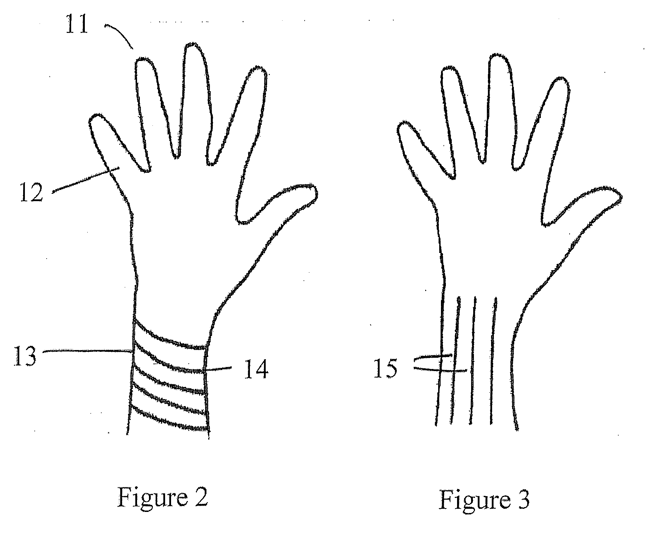

Referring next to Figure 2, there is shown a surgical glove 11 having a hand proton 12 and a cuff portion 13. The glove is manufactured in conventional manner using a former or mould onto which elastomeric material, such as latex, is sprayed and dried to form a thin film which is subsequently peeled off of the mould.

The cuff portion 13 of the glove 11 includes reinforcing beads 14, in the form of a spiral bead of thickened elastomeric material, in particular latex, which is applied to the outer most surface of the glove when it is still on the former (which, in use, forms the inner surface of the glove which engages against the skin of the wearer) by spraying or extrusion or the like. These reinforcing beads act to stiffen the cuff portion of the glove, which thereby acts to prevent slip or roll down of the cuff when in use, or so called scrunching. Preferably, the glove if formed of latex having a thickness of 0.25mm and the reinforcing beads are added to an additional thickness of 1mm.

In an alternative configuration shown in Figure 3, the reinforcing beading is applied in longitudinally extending strips 15, distributed around the circumference of the wrist of the glove. However, other configurations are also possible, such as a pattern of nodules or dots or the like.

## Claims

1. A glove (11) comprising a hand portion (12) and a wrist portion (13) extending from the hand portion (12) and terminating in a hand insertion opening, wherein the wrist portion (13) of the glove (11), at least in the region of said opening, is reinforced with elastomeric material (14) applied to a surface which, in use, will form an inside surface of the glove.

2. A glove according to claim 1, wherein the elastomeric material extends in longitudinal (15) or spiral bands (14) from the cuff opening.

3. A glove according to claim 1, wherein the elastomeric material is applied in a pattern of dots.

4. A glove according to any of claims 1 to 3, wherein the same material is used for the main body of the glove (11) and for the reinforcing material (14).

5. A method of manufacturing a glove comprising the steps of coating a former with material to form the glove (11), and selectively applying additional elastomeric material (14) to areas of the glove material on the former which form a cuff portion (14) of the glove so as to form reinforcing and gripping means (14) on said cuff portion (13).

6. A method according to claim 5, wherein the elastomeric material is gun-fired onto the glove in the required pattern when the glove (11) is on the former.
